# EUROPEAN PATENT APPLICATION

(11) **EP 3 118 197 A1**
(43) Date of publication of application: **18.01.2017**
(21) Application number: 15761578.2
(22) Date of filing: 11.03.2015
(51) Int. Cl.: C07D 333/40

(54) **PRODUCTION METHOD FOR HETEROARYLCARBOXYLIC ACID ESTER DERIVATIVE, PRODUCTION INTERMEDIATE THEREOF, AND CRYSTAL**

(30) Priority: 11.03.2014 JP 2014048019
(71) Applicant: EA Pharma Co., Ltd., Tokyo 104-0042 (JP)
(72) Inventor: YAMADA, Tatsuhiro, Kawasaki-shi, Kanagawa 210-8681 (JP); TATARA, Akinori, Kawasaki-shi. Kanagawa 210-8681 (JP); TAKASHITA, Ryuta, Kawasaki-shi, Kanagawa 210-8681 (JP); KODAMA, Riho, Kawasaki-shi, Kanagawa 210-8681 (JP); OOKUMA, Kazutaka, Kawasaki-shi, Kanagawa 210-8681 (JP)
(74) Representative: Nicholls, Kathryn Margaret
(86) International application number: PCT/JP2015/057177
(87) International publication number: WO 2015/137407

(57) **Abstract**

Provision of a production method of compound (VI) useful as a therapeutic drug for diabetes, its intermediate, and its crystal. A production method of compound (VI) or a salt thereof, including (d) a step of reacting compound (II) with an acid halogenating agent to give an acid halide, (e) a step of reacting the acid halide with compound (IV) in the presence of a base, and crystallizing compound (V) or a salt thereof from the reaction system, and (f) a step of subjecting the compound (V) to reductive deprotection in the presence of a metal catalyst, and crystallizing compound (VI) or a salt thereof from the reaction system: 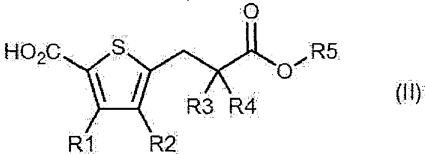 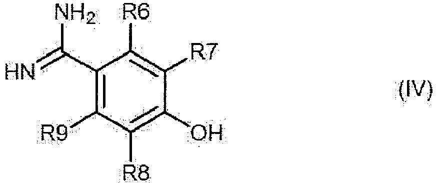 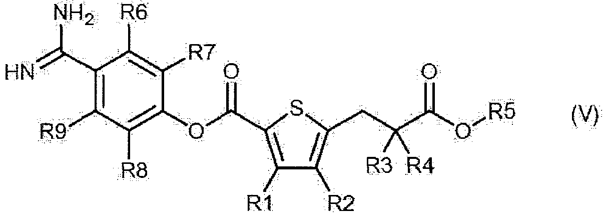 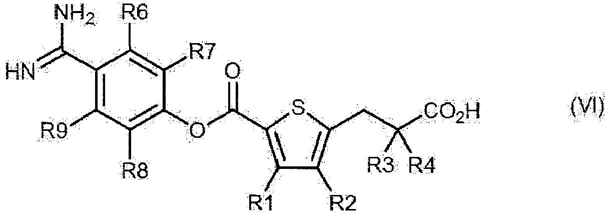 wherein R5 is an aralkyl group optionally having a substituent(s), an aralkyloxymethyl group optionally having a substituent(s), or a heteroarylalkyl group optionally having a substituent(s).

## Description

### Technical Field

The present invention relates to a novel production method of a heteroarylcarboxylic acid ester derivative, its intermediate, and its crystal. More particularly, the present invention relates to an efficient production method of a heteroarylcarboxylic acid ester derivative as a therapeutic drug for diabetes or an intermediate therefor, and an intermediate useful for such production method. The present invention also relates to a crystal of a heteroarylcarboxylic acid ester derivative useful as a therapeutic drug for diabetes.

### Background Art

At present, insulin secretagogue (sulfonylurea), glucose absorption inhibitor (α-glucosidase inhibitor), insulin sensitizer (biguanide, thiazolidine derivative) and the like are clinically used as therapeutic drugs for diabetes. However, all of them still have problems in that they are accompanied by side effects such as hypoglycemia, diarrhea, lactic acidosis, edema and the like, show insufficient effect, and the like. As a new treatment or prophylactic drug for diabetes that satisfies clinical needs, patent document 1 and patent document 2 disclose heteroarylcarboxylic acid ester derivatives encompassed by the following formula (I), and it has been reported that representative compounds thereof show a superior blood glucose elevation suppressing effect in diabetes animal models.

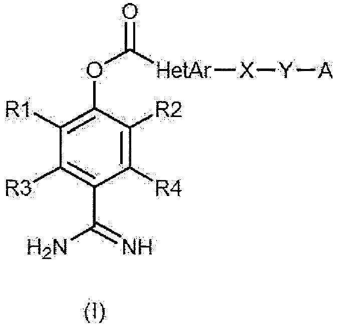

In patent document 1, a method shown by the following scheme is disclosed as a general synthesis method of a heteroarylcarboxylic acid ester derivative encompassed by the formula (I) (refer to patent document 1 for symbols in the formula).

A heteroarylcarboxylic acid ester derivative (F) of the formula (I) wherein X is a lower alkylene group or a lower alkenylene group, A is -OR5, and R5 is a lower alkyl group can be produced as follows.

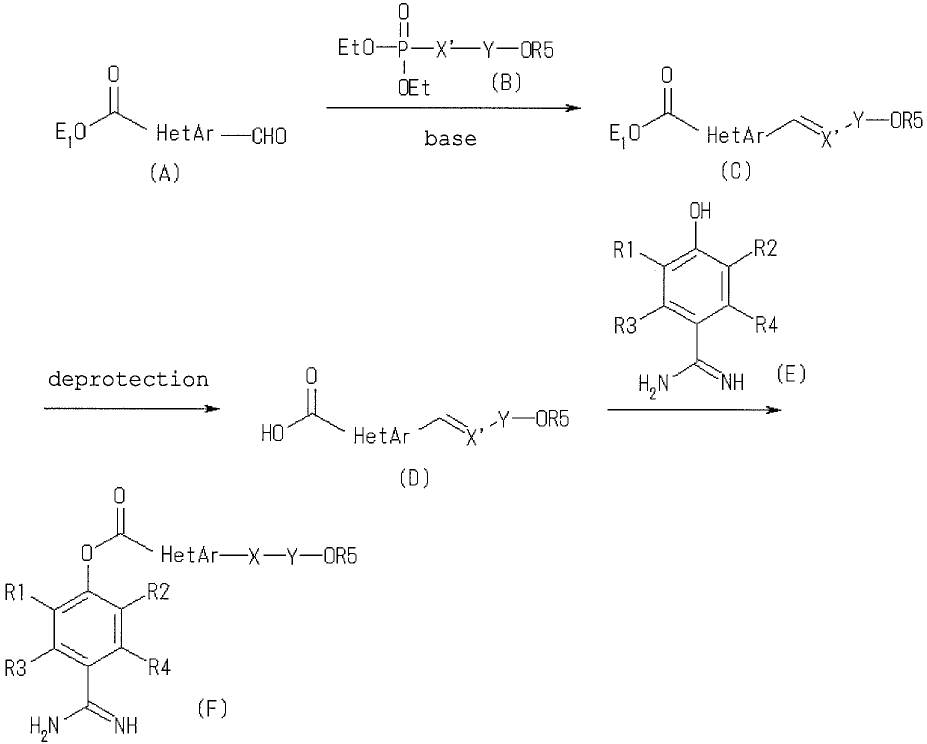

An object heteroarylcarboxylic acid ester derivative (F) wherein X is a lower alkenylene group can be produced by esterification of a carboxylic acid derivative (D) and an amidinophenol derivative (E), and a heteroarylcarboxylic acid ester derivative (F) wherein X is a lower alkylene group can be produced by a step of treating with a catalyst such as 10% palladium/carbon, in a solvent that does not adversely influence this reaction, such as methanol, ethanol, ethyl acetate and the like, under a hydrogen atmosphere at some stage of the production step.

A known method can be applied to the esterification reaction and, for example, (1) a method using an acid halide, (2) a method using a condensing agent and the like can be mentioned.

The (1) method using an acid halide is performed by reacting a carboxylic acid with thionyl chloride, oxalyl chloride and the like in a solvent that does not adversely influence this reaction such as dichloromethane and the like or without solvent in the presence or absence of a catalyst such as N,N-dimethylformamide and the like to give an acid chloride, and reacting the obtained acid chloride with an alcohol in a solvent that does not adversely influence this reaction such as dichloromethane, tetrahydrofuran and the like, in the presence of a base such as pyridine and triethylamine.

The (2) method using a condensing agent is performed by reacting a carboxylic acid and an alcohol in a solvent that does not adversely influence this reaction such as tetrahydrofuran, N,N-dimethylformamide, dichloromethane and the like, in the presence or absence of a base such as pyridine, triethylamine and the like by using a condensing agent such as 1-ethyl-3-(3'-dimethylaminopropyl)carbodiimide (WSC) or 1,3-dicyclohexylcarbodiimide and the like.

A heteroarylcarboxylic acid ester derivative (i) of the formula (I) wherein A is -OR5, and R5 is a hydrogen atom can be produced by allowing an ester derivative (H) obtained by using a Wittig reagent (G) (wherein E₂ is a protecting group such as methyl group, ethyl group, isopropyl group, tert-butyl group, benzyl group and the like) instead of a Wittig reagent (B) to undergo, for example, hydrolysis by a base such as sodium hydroxide and the like, hydrolysis by an acid such as hydrochloric acid, trifluoroacetic acid and the like or deprotection by a treatment with 10% palladium/carbon etc. under a hydrogen atmosphere, and the like.

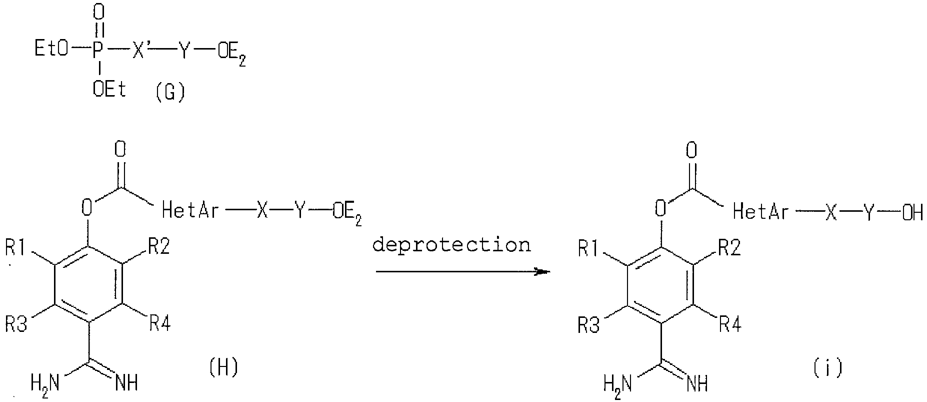

Also, patent document 2 provides a similar description of a general synthesis method of a heteroarylcarboxylic acid ester derivative encompassed by the formula (I).

However, in the Examples of patent document 1 and patent document 2, column chromatography is used for an isolation operation of ester derivative (F) and (i), and the yield thereof is not appropriate for an industrial process. Thus, while a heteroarylcarboxylic acid ester derivative encompassed by the formula (I) is expected as a useful therapeutic drug for diabetes, economic efficiency and productivity are poor by a conventional production method, and a new method capable of industrial production with good efficiency is desired.

### Document List

### Patent Documents

patent document 1: WO 2011/071048
patent document 2: WO 2013/187533

### Summary of the Invention

### Problems to be Solved by the Invention

An industrial production method for producing a heteroarylcarboxylic acid ester derivative encompassed by the formula (I) in a better yield with better quality than conventional methods is desired.

### Means of Solving the Problems

To solve the above-mentioned problem, intensive studies have been conducted and an industrial production method for producing a heteroarylcarboxylic acid ester derivative shown below and a novel intermediate useful for preparing the derivative have been found, which resulted in the completion of the present invention.

Accordingly, the present invention provides a production method of a heteroarylcarboxylic acid ester derivative represented by the following formula (VI) suitable for industrialization and an intermediate useful for the production thereof.

In addition, patent document 2 describes 3-[5-(4-carbamimidoyl-2-fluorophenoxy)carbonyl-2-thienyl]-2,2-dimethylpropanoic acid trifluoroacetate. The trifluoroacetate is obtained from a reaction mixture after purification by high performance liquid chromatography. The present inventors have succeeded in producing a crystal of 3-[5-(4-carbamimidoyl-2-fluorophenoxy)carbonyl-2-thienyl]-2,2-dimethylpropanoic acid hydrochloride, and found that the crystal is superior in preservation stability.

The present invention relates to the following.
[1] A method for producing a heteroarylcarboxylic acid ester derivative represented by the formula (VI) or a salt thereof: 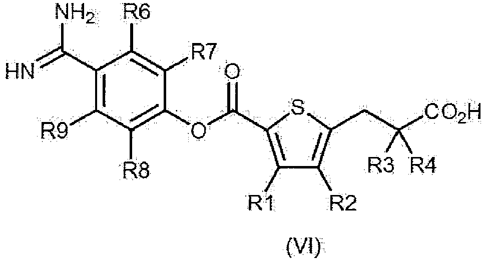
   wherein R1 and R2 are the same or different and each is independently a hydrogen atom or a lower alkyl group optionally having a substituent(s),
   R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto, and
   R6, R7, R8 and R9 are the same or different and each is independently a hydrogen atom or a halogen atom, the method comprising the following steps (d) to (f):
      (d) a step of reacting a compound represented by the formula (II) with an acid halogenating agent to give an acid halide represented by the formula (III): 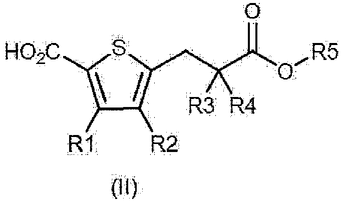 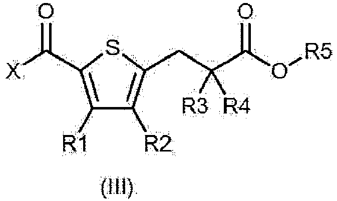
         wherein R5 is an aralkyl group optionally having a substituent(s), an aralkyloxymethyl group optionally having a substituent(s), or a heteroarylalkyl group optionally having a substituent(s),
         X is a halogen atom, and
         other symbols are as defined above,
      (e) a step of reacting the acid halide represented by the formula (III) with a compound represented by the formula (IV) in the presence of a base, and
         crystallizing a compound represented by the formula (V) or a salt thereof from the reaction system: 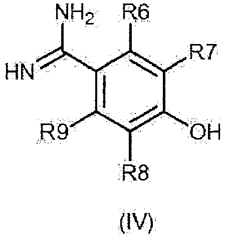 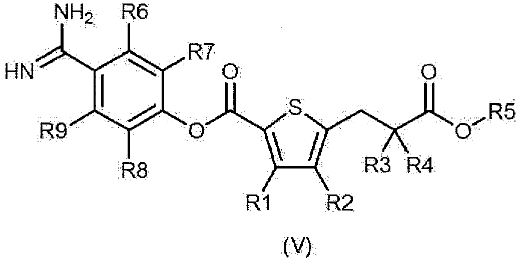 wherein each symbol is as defined above, and
      (f) a step of subjecting the compound represented by the formula (V) to reductive deprotection in the presence of a metal catalyst, and crystallizing a heteroarylcarboxylic acid ester derivative represented by the formula (VI) or a salt thereof from the reaction system.
[2] A method for producing a heteroarylcarboxylic acid ester derivative represented by the formula (VI) or a salt thereof: 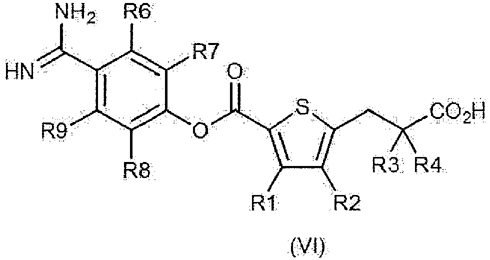
   wherein R1 and R2 are the same or different and each is independently a hydrogen atom or a lower alkyl group optionally having a substituent(s),
   R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto, and
   R6, R7, R8 and R9 are the same or different and each is independently a hydrogen atom or a halogen atom, the method comprising the following steps (g) to (i):
      (g) a step of reacting a compound represented by the formula (II) with thionyl chloride or oxalyl chloride to give an acid chloride represented by the formula (IX): 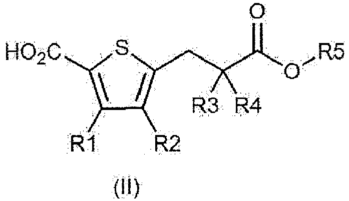 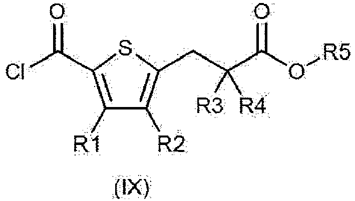 wherein R5 is an aralkyl group optionally having a substituent(s), an aralkyloxymethyl group optionally having a substituent(s), or a heteroarylalkyl group optionally having a substituent(s), and
         other symbols are as defined above,
      (h) a step of reacting the acid chloride represented by the formula (IX) with a compound represented by the formula (IV) in the presence of an organic base, and crystallizing a compound represented by the formula (V) or a salt thereof from the reaction system: 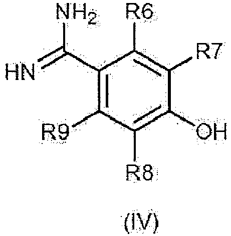 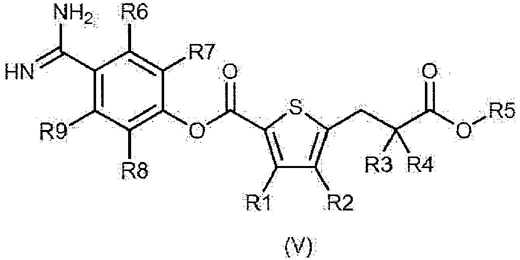 wherein each symbol is as defined above, and
      (i) a step of subjecting the compound represented by the formula (V) to hydrogenolysis in the presence of a palladium catalyst, and crystallizing a heteroarylcarboxylic acid ester derivative represented by the formula (VI) or a salt thereof from the reaction system.
[3] The method of the aforementioned [1] or [2], wherein R5 is an aralkyl group optionally having a substituent(s).
[4] The method of the aforementioned [1] or [2], wherein R1 and R2 are hydrogen atoms, and R5 is an aralkyl group optionally having a substituent(s).
[5] The method of the aforementioned [1] or [2], wherein R3 and R4 are methyl groups, and
   R5 is an aralkyl group optionally having a substituent(s).
[6] The method of the aforementioned [1] or [2], wherein R1 and R2 are hydrogen atoms,
   R3 and R4 are methyl groups, and
   R5 is an aralkyl group optionally having a substituent(s).
[7] The method of the aforementioned [1] or [2], wherein R1 and R2 are hydrogen atoms,
   R3 and R4 are methyl groups,
   R5 is a benzyl group,
   R8 is a fluorine atom, and
   R6, R7 and R9 are hydrogen atoms.
[8] A method for producing a heteroarylcarboxylic acid ester derivative represented by the formula (X) or a salt thereof: 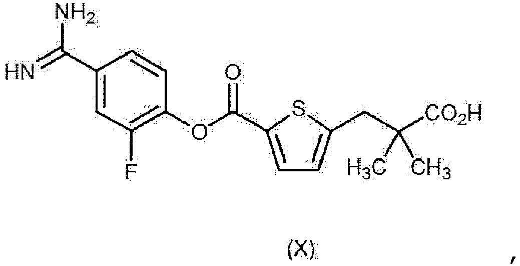 the method comprising the following steps (j) to (1) :
   (j) a step of reacting a compound represented by the formula (VII) with thionyl chloride to give an acid chloride represented by the formula (XI): 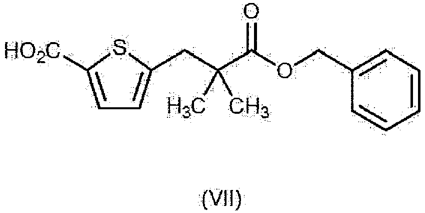 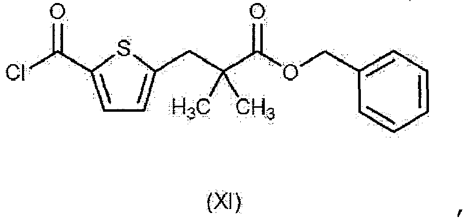
   (k) a step of reacting the acid chloride represented by the formula (XI) with a compound represented by the formula (XII) in the presence of pyridine, and crystallizing a compound represented by the formula (VIII) or a salt thereof from the reaction system: 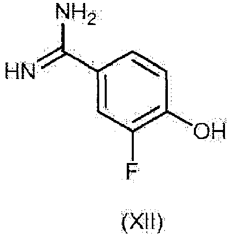 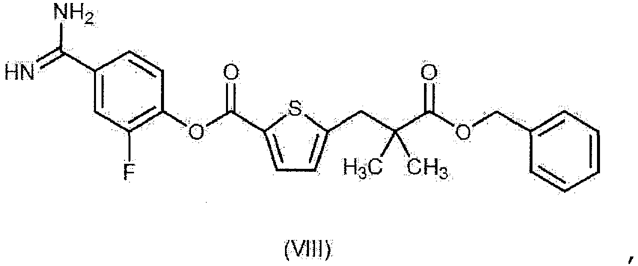 and
   (l) a step of subjecting the compound represented by the formula (VIII) to hydrogenolysis in the presence of a palladium hydroxide catalyst, and crystallizing a heteroarylcarboxylic acid ester derivative represented by the formula (X) or a salt thereof from the reaction system.
[9] A compound represented by the following formula (II), or a chemically acceptable salt thereof: 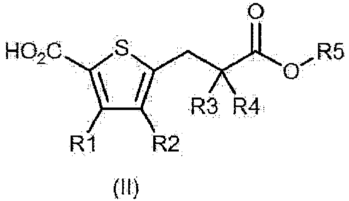
   wherein R1 and R2 are the same or different and each is independently a hydrogen atom or a lower alkyl group optionally having a substituent(s),
   R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto, and
   R5 is an aralkyl group optionally having a substituent(s), an aralkyloxymethyl group optionally having a substituent(s), or a heteroarylalkyl group optionally having a substituent(s).
[10] The compound of the aforementioned [9], wherein, in the formula (II), R1 and R2 are hydrogen atoms,
   R3 and R4 are the same or different and each is independently a methyl group, an ethyl group or a propyl group (excluding when R3 and R4 are ethyl groups), or R3 and R4 form a cyclopropane ring, a cyclobutane ring or a cyclopentane ring together with a carbon atom bonded thereto,
   or a chemically acceptable salt thereof.
[11] The compound of the aforementioned [9] or [10], wherein, in the formula (II), R3 and R4 are methyl groups, or a chemically acceptable salt thereof.
[12] A compound represented by the following formula (VII), or a chemically acceptable salt thereof: 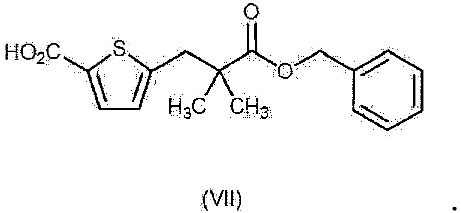
[13] A compound represented by the following formula (V), or a chemically acceptable salt thereof: 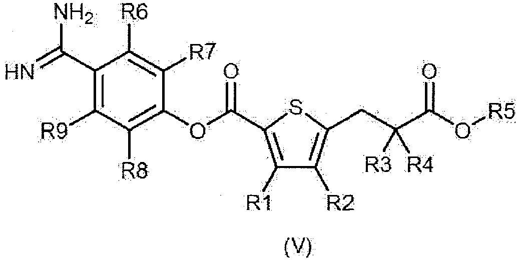
   wherein R1 and R2 are the same or different and each is independently a hydrogen atom or a lower alkyl group optionally having a substituent(s),
   R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto,
   R5 is an aralkyl group optionally having a substituent(s), an aralkyloxymethyl group optionally having a substituent(s), or a heteroarylalkyl group optionally having a substituent(s), and R6, R7, R8 and R9 are the same or different and each is independently a hydrogen atom or a halogen atom.
[14] The compound of the aforementioned [13], wherein, in the formula (V), R1 and R2 are hydrogen atoms, and R3 and R4 are the same or different and each is independently a methyl group, an ethyl group or a propyl group (excluding when R3 and R4 are ethyl groups), or R3 and R4 form a cyclopropane ring, a cyclobutane ring or a cyclopentane ring together with a carbon atom bonded thereto,
   or a chemically acceptable salt thereof.
[15] The compound of the aforementioned [13] or [14], wherein, in the formula (V), R3 and R4 are methyl groups, or a chemically acceptable salt thereof.
[16] A compound represented by the following formula (VIII), or a chemically acceptable salt thereof: 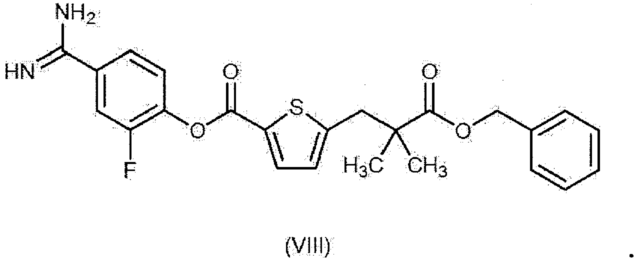
[17] A crystal of 3-[5-(4-carbamimidoyl-2-fluorophenoxy)carbonyl-2-thienyl]-2,2-dimethylpropanoic acid hydrochloride showing an X-ray powder diffraction pattern comprising peaks at least at 19.6°, 23.1°, 24.0° and 24.2°(2θ).

### Effect of the Invention

The present invention provides a production method and a novel intermediate suitable for the mass synthesis of a heteroarylcarboxylic acid ester derivative. Since a compound of the formula (II) wherein R5 is an aralkyl group optionally having a substituent(s), an aralkyloxymethyl group optionally having a substituent(s), or a heteroarylalkyl group optionally having a substituent(s) is used in the production method of the present invention, a compound of the formula (V) and a compound of the formula (VI) can be crystallized from the reaction system, and the resultant product can be conveniently isolated and purified by filtration and separation. Using the production method of the present invention, the object compound, a heteroarylcarboxylic acid ester derivative, can be produced in a high yield and with high purity.

The crystal of 3-[5-(4-carbamimidoyl-2-fluorophenoxy)carbonyl-2-thienyl]-2,2-dimethylpropanoic acid hydrochloride of the present invention can be purified by crystallization instead of purification by liquid chromatography. Since the crystal of the present invention has properties excellent in preservation stability, it is useful as a crystalline form of a drug substance for pharmaceutical products.

### Brief Description of the Drawings

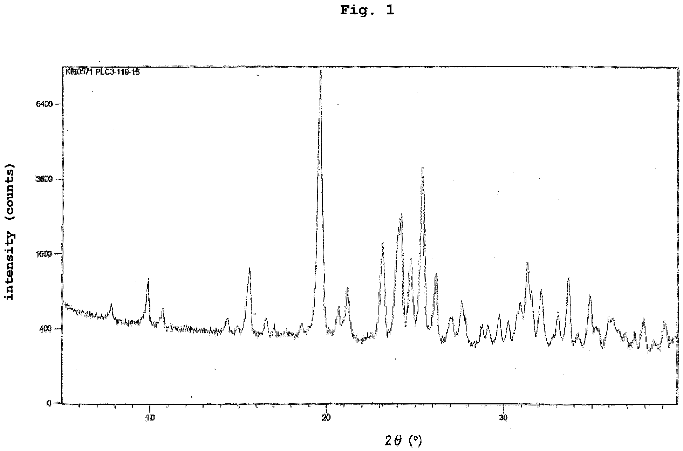

Fig. 1 shows a powder X-ray diffraction pattern of the crystal of the present invention obtained by the method of Example 3.

### Description of Embodiments

In the present specification, the term "optionally having a substituent(s)" means "being substituted or unsubstituted". Unless otherwise specified, the position and number of the substituents may be any, and are not particularly limited. The number of the substituents is preferably 1 to 5, more preferably 1 to 3. When substituted by two or more substituents, the substituents may be the same or different. Examples of the substituent include oxo group, halogen atom, cyano group, formyl group, phenyl group, lower acyl group, lower alkoxyl group, lower alkylthio group, lower acyloxy group, lower acylamino group, lower alkoxycarbonyl group, carbamoyl group, lower alkylcarbamoyl group, lower alkylsulfonylamino group, sulfamoyl group and the like.

The "lower alkyl group" is a straight chain or branched chain or cyclic alkyl group having a carbon number of 1 to 6. For example, methyl group, ethyl group, n-propyl group, n-butyl group, n-pentyl group, n-hexyl group, isopropyl group, isobutyl group, sec-butyl group, tert-butyl group, isopentyl group, tert-pentyl group, neopentyl group, 2-pentyl group, 3-pentyl group, 2-hexyl group, cyclopropyl group, cyclobutyl group, cyclopentyl group and the like can be mentioned.

The "aryl group" is a monocyclic to tricyclic aryl group having a carbon number of 6 to 14, and specifically, phenyl group, naphthyl group, anthryl group, phenanthryl group, biphenyl group and the like can be mentioned.

The "aralkyl group" is the aforementioned lower alkyl group substituted with an aryl group(s), and the lower alkyl group and the aryl group may be joined to form a ring. Specifically, benzyl group, diphenylmethyl group, trityl group, phenethyl group, 3-phenylpropyl group, 2-phenylpropyl group, 4-phenylbutyl group, biphenylmethyl group, naphthylmethyl group and the like can be mentioned. When a ring is formed, benzocyclobutenyl group, indanyl group and the like can be mentioned.

The "aralkyl group" is preferably a C₆₋₁₄ aryl-C₁₋₆ alkyl group, more preferably, a phenyl-C₁₋₆ alkyl group, further preferably, benzyl group.

The "heteroaryl group" is a 5- to 14-membered monocyclic to tricyclic heterocyclic group containing, as a ring atom, 1 to 4 hetero atoms selected from an oxygen atom, a sulfur atom and a nitrogen atom. Any carbon atom as a ring atom may be substituted by an oxo group, and a sulfur atom or a nitrogen atom may be oxidized to form an oxide. In addition, it may be condensed with a benzene ring. For example, pyridyl group, pyridazinyl group, pyrimidinyl group, pyrazinyl group, furyl group, thienyl group, pyrrolyl group, isoxazolyl group, oxazolyl group, isothiazolyl group, thiazolyl group, pyrazolyl group, imidazolyl group, oxadiazolyl group, thiadiazolyl group, triazolyl group, tetrazolyl group, benzofuranyl group, benzothienyl group, indolyl group, isoindolyl group, benzoxazolyl group (= benzoxazolyl group), benzothiazolyl group, benzimidazolyl group (= benzimidazolyl group), indazolyl group, benzisoxazolyl group, benzisothiazolyl group, benzofurazanyl group, benzothiadiazolyl group, purinyl group, quinolinyl group, isoquinolyl group, cinnolinyl group, phthalazinyl group, quinazolinyl group, quinoxalinyl group, pteridinyl group, imidazooxazolyl group, imidazothiazolyl group, imidazoimidazolyl group, dibenzofuranyl group, dibenzothienyl group, carbazolyl group, acridinyl group, pyrrolidinyl group, pyrazolidinyl group, imidazolidinyl group, pyrrolinyl group, pyrazolinyl group, imidazolinyl group, tetrahydrofuranyl group, tetrahydrothiophenyl group, thiazolidinyl group, piperidinyl group, piperazinyl group, quinuclidinyl group, tetrahydropyranyl group, tetrahydrothiopyranyl group, morpholinyl group, thiomorpholinyl group, dioxolanyl group, homopiperidinyl group, homopiperazinyl group, indolinyl group, isoindolinyl group, chromanyl group, isochromanyl group, tetrahydronaphthyridinyl group, azaindolyl group and the like can be mentioned. Preferably, pyridyl group, thiadiazolyl group, imidazolyl group, tetrazolyl group, piperidinyl group, piperazinyl group, thiazolidinyl group and the like can be mentioned.

The "heteroarylalkyl group" is the aforementioned lower alkyl group substituted with the aforementioned heteroaryl group and, for example, picolyl group (pyridylmethyl group) can be mentioned.

The "aralkyloxymethyl group" is the aforementioned "aralkyl group" wherein an oxygen atom is bonded to a lower alkyl group moiety, and a methylene group is further bonded to the oxygen atom. For example, benzyloxymethyl group can be mentioned.

The "halogen atom" is, for example, a fluorine atom, a chlorine atom, a bromine atom, an iodine atom and the like.

The "C₃₋₈ cycloalkane ring" is a cycloalkane ring having a carbon number of 3 to 8. For example, cyclopropane ring, cyclobutane ring, cyclopentane ring, cyclohexane ring, cycloheptane ring, and cyclooctane ring can be mentioned.

The "lower acyl group" is an acyl group having a straight chain or branched chain or cyclic alkyl group or alkenyl group having a carbon number of 1 to 6. For example, acetyl group, propionyl group, butyryl group, isobutyryl group, valeryl group, isovaleryl group, pivaloyl group, hexanoyl group, acryloyl group, methacryloyl group, crotonoyl group, isocrotonoyl group, cyclopropanoyl group, cyclobutanoyl group, cyclopentanoyl group and cyclohexanoyl group and the like can be mentioned.

The "lower alkoxyl group" is an alkoxyl group having a straight chain or branched chain or cyclic alkyl group having a carbon number of 1 to 6. For example, methoxy group, ethoxy group, n-propoxy group, n-butoxy group, n-pentyloxy group, n-hexyloxy group, isopropoxy group, isobutoxy group, sec-butoxy group, tert-butoxy group, cyclopropyloxy group, cyclobutyloxy group, cyclopentyloxy group and cyclohexyloxy group can be mentioned.

The "lower alkylthio group" is an alkylthio group having a straight chain or branched chain or cyclic alkyl group having a carbon number of 1 to 6. For example, methylthio group, ethylthio group, n-propylthio group, isopropylthio group, n-butylthio group, isobutylthio group, sec-butylthio group, tert-butylthio group, cyclopropylthio group, cyclobutylthio group, cyclopentylthio group, cyclobutylthio group and the like can be mentioned.

The "lower acyloxy group" is the aforementioned "lower acyl group" wherein an oxygen atom is bonded to a carbon of the carbonyl moiety. For example, acetyloxy group, propionyloxy group, butyryloxy group, isobutyryloxy group, valeryloxy group, isovaleryloxy group, pivaloyloxy group, hexanoyloxy group, acryloyloxy group, methacryloyloxy group, crotonoyloxy group, isocrotonoyloxy group and the like can be mentioned.

The "lower acylamino group" is the aforementioned "lower acyl group" wherein a nitrogen atom is bonded to a carbon of the carbonyl moiety. For example, acetylamino group, propionylamino group, butyrylamino group, isobutyrylamino group, valerylamino group, isovalerylamino group, pivaloylamino group, hexanoylamino group, acryloylamino group, methacryloylamino group, crotonoylamino group, isocrotonoylamino group and the like can be mentioned.

The "lower alkoxycarbonyl group" is a carbonyl group having the aforementioned "lower alkoxyl group". For example, methoxycarbonyl group, ethoxycarbonyl group, propoxycarbonyl group, isopropoxycarbonyl group, n-butoxycarbonyl group, isobutoxycarbonyl group, sec-butoxycarbonyl group, tert-butoxycarbonyl group and the like can be mentioned.

The "lower alkylcarbamoyl group" is a group wherein a nitrogen atom of the "lower alkylamino group" or "cyclic amino group" and a carbon atom of a carbonyl group are bonded. For example, N-methylcarbamoyl group, N-ethylcarbamoyl group, N,N-dimethylcarbamoyl group, 1-pyrrolidinylcarbonyl group, 1-piperidinylcarbonyl group, 4-morpholinylcarbonyl group and the like can be mentioned.

The "lower alkylamino group" is an amino group mono- or di-substituted by the aforementioned "lower alkyl group". For example, methylamino group, ethylamino group, propylamino group, isopropylamino group, dimethylamino group, diethylamino group, dipropylamino group, diisopropylamino group, ethylmethylamino group and the like can be mentioned.

The "cyclic amino group" is a saturated or unsaturated cyclic amino group having a carbon number of 2 to 7, which may further have one or more hetero atoms such as oxygen atom, sulfur atom and the like in the ring. For example, 1-pyrrolidinyl group, 1-piperidinyl group, 4-morpholinyl group and the like can be mentioned.

The "lower alkylsulfonylamino group" is a sulfonyl group wherein a nitrogen atom is bonded to a sulfur atom to which the aforementioned "lower alkyl group" is bonded. For example, methylsulfonylamino group, ethylsulfonylamino group, propylsulfonylamino group, isopropylsulfonylamino group, n-butylsulfonylamino group, isobutylsulfonylamino group and the like can be mentioned.

In the aforementioned formulas, preferable embodiments are as follows.

R1 and R2 are the same or different and each is independently a hydrogen atom or a lower alkyl group optionally having a substituent(s). Preferably, R1 and R2 are hydrogen atoms.

R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto. Preferably, R3 and R4 are the same or different and each is independently a methyl group, an ethyl group or a propyl group (excluding when R3 and R4 are ethyl groups), or R3 and R4 form a cyclopropane ring, a cyclobutane ring or a cyclopentane ring together with a carbon atom bonded thereto. More preferably, R3 and R4 are methyl groups.

R5 is an aralkyl group optionally having a substituent(s), an aralkyloxymethyl group optionally having a substituent(s), or a heteroarylalkyl group optionally having a substituent(s). Preferably, R5 is a benzyl group or a benzyloxymethyl group. More preferably, R5 is a benzyl group.

R6, R7, R8 and R9 are the same or different and each is independently a hydrogen atom or a halogen atom. Preferably, R8 is a fluorine atom, and the R6, R7 and R9 are hydrogen atoms.

X is a halogen atom. Preferably, X is a chlorine atom.

When the compound of the present invention can form a salt, a pharmaceutically acceptable salt is preferable. Examples of such pharmaceutically acceptable salts for a compound having an acidic group such as a carboxyl group and the like include ammonium salt, salts with alkali metals such as sodium, potassium and the like, salts with alkaline earth metals such as calcium and the like, magnesium salt, aluminum salt, zinc salt, salts with organic amines such as triethylamine, ethanolamine, morpholine, pyrrolidine, piperidine, piperazine, dicyclohexylamine and the like, and salts with basic amino acids such as arginine, lysine and the like. Examples of such pharmaceutically acceptable salts for a compound having a basic group include salts with inorganic acids such as hydrochloric acid, sulfuric acid, phosphoric acid, nitric acid, hydrobromic acid and the like, salts with organic carboxylic acids such as acetic acid, citric acid, benzoic acid, maleic acid, fumaric acid, tartaric acid, succinic acid, trifluoroacetic acid, tannic acid, butyric acid, hibenzic acid, pamoic acid, enanthic acid, decanoic acid, teoclic acid, salicylic acid, lactic acid, oxalic acid, mandelic acid, malic acid and the like, and salts with organic sulfonic acids such as methanesulfonic acid, benzenesulfonic acid, p-toluenesulfonic acid and the like.

As a salt used in the present invention, a chemically acceptable salt can be mentioned in addition to those recited as the above-mentioned pharmaceutically acceptable salt, and a salt with a chemically acceptable acid and a salt with a chemically acceptable base are included.

As a salt with a chemically acceptable acid to be used in the present invention, salts with inorganic acids (e.g., hydrochloric acid, sulfuric acid, phosphoric acid, nitric acid, hydrobromic acid, etc.), organic carboxylic acids (e.g., carbonic acid, acetic acid, citric acid, benzoic acid, maleic acid, fumaric acid, tartaric acid, succinic acid, trifluoroacetic acid, tannic acid, butyric acid, decanoic acid, salicylic acid, lactic acid, oxalic acid, mandelic acid, malic acid, etc.), organic sulfonic acids (e.g., methanesulfonic acid, p-toluenesulfonic acid, benzenesulfonic acid, etc.) and the like can be mentioned.

As a salt with a chemically acceptable base, alkali metal salts (e.g., sodium salt, potassium salt, lithium salt, etc.), alkaline earth metal salts (e.g., calcium salt, barium salt, etc.), metal salts (e.g., magnesium salt, aluminum salt, etc.) and the like can be mentioned.

The compound of the present invention also includes solvates of the compound such as hydrate, alcohol adduct and the like. As the alcohol adduct, methanol solvate, ethanol solvate, isopropyl alcohol solvate and the like can be mentioned.

A production method of a heteroarylcarboxylic acid ester derivative represented by the formula (VI) is shown below.

The present invention is a method for producing a compound represented by the formula (VI) or a salt thereof, comprising the following steps (a) to (c),

(a) a step of reacting a compound represented by the formula (II) with an acid halogenating agent to give an acid halide represented by the formula (III): 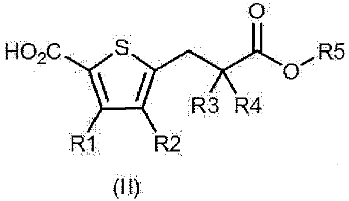 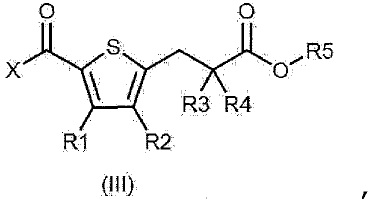 (b) a step of reacting a compound represented by the formula (III) with a compound represented by the formula (IV) to give a compound represented by the formula (V) or a salt thereof: 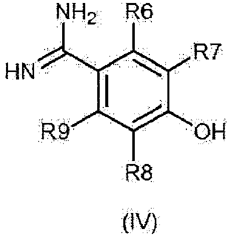 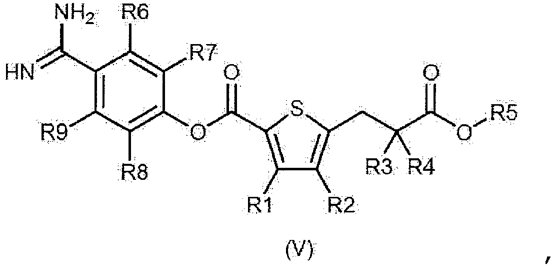 and
(c) a step of subjecting the compound represented by the formula (V) to reductive deprotection in the presence of a metal catalyst to give a heteroarylcarboxylic acid ester derivative represented by the formula (VI) or a salt thereof:

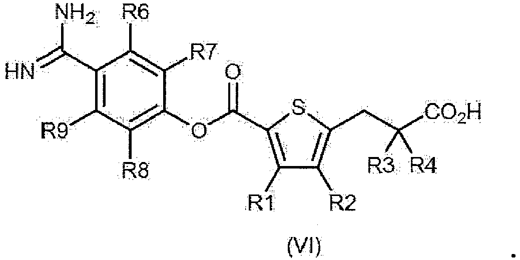

A preferable embodiment is described in more detail in the following.

### Step (a)

As a reaction solvent for acid halogenation, dichloromethane, N-methylpyrrolidone, acetonitrile and the like can be mentioned, and acetonitrile is preferable.

As the acid halogenating agent, thionyl chloride or oxalyl chloride is preferable.

While the amount of the reaction solvent to be used is not particularly limited, about 3 ml to about 5 ml is preferable per 1 g of the compound of the formula (II).

The amount of the acid halogenating agent to be used is generally about 1 mol to about 1.5 mol, preferably about 1.3 mol, per 1 mol of the compound of the formula (II).

The reaction temperature is between 0°C and 45°C, preferably 10°C to 30°C. The reaction time is generally about 3 to about 4 hr.

### Step (b)

A diester derivative represented by the formula (V) is obtained by reacting an acid halide (III) obtained in step (a) with an amidinophenol derivative (IV) preferably in the presence of a base.

While the order of charging of the starting materials and reagents is not particularly limited, it is preferable from the aspect of operability to add an acid halide of the formula (III) to a mixture of an amidinophenol derivative of the formula (IV) and a base.

It is preferable to add the reaction mixture obtained in step (a) to a mixture of the amidinophenol derivative of the formula (IV) and the base, without isolating the acid halide (III) obtained in step (a).

As the base, organic bases such as pyridine, triethylamine, diisopropylethylamine, lutidine and the like can be mentioned. As the base, pyridine is preferable. The amount of the base to be used is generally about 2 mol to about 6 mol, preferably about 3 mol, per 1 mol of acid halide (III).

The amount of the amidinophenol derivative (IV) to be used is generally about 1 mol to about 1.5 mol, preferably about 1.1 mol, per 1 mol of acid halide (III).

The reaction temperature is between -60°C and 30°C, preferably -25°C to 0°C. The reaction time is generally about 1 hr.

A diester derivative (V) can be crystallized as a TFA salt from the reaction system by adding an aqueous trifluoroacetic acid (TFA) solution dropwise to the reaction mixture, and can be isolated and purified by filtration and separation. The amount of TFA to be used is generally about 2 mol to about 6 mol, preferably about 3 mol, per 1 mol of acid halide (III).

The temperature of dropwise addition and crystallization is between 0°C and 20°C, preferably not more than 10°C. The crystallization time is generally about 1 hr.

The precipitated diester derivative (V) or a salt thereof can be conveniently isolated and purified by filtration and separation.

### Step (c)

A heteroarylcarboxylic acid ester derivative represented by the formula (VI) is obtained by reductive deprotection (hydrogenolysis) of the diester derivative (V) obtained in step (b) preferably in the presence of a metal catalyst.

As the metal catalyst, palladium catalyst (e.g., palladium carbon, palladium hydroxide carbon, palladium oxide), platinum catalyst (e.g., platinum carbon, platinum oxide), rhodium catalyst (e.g., rhodium carbon), ruthenium catalyst (e.g., ruthenium carbon) and the like can be mentioned. As the metal catalyst, a palladium catalyst is preferable, and a palladium hydroxide catalyst (e.g., palladium hydroxide carbon) is more preferable.

The amount of the metal catalyst to be used is generally about 0.2 mol to about 0.5 mol, preferably about 0.3 mol to about 0.35 mol, per 1 mol of diester derivative (V).

As the reaction solvent, a mixed solvent of alcohol such as methanol, ethanol, isopropyl alcohol and the like and water can be mentioned, and a mixed solvent of isopropyl alcohol and water is preferable. The ratio of isopropyl alcohol and water is preferably isopropyl alcohol:water = 1:1 to 6:1, more preferably 4:1, in a volume ratio. While the amount of the reaction solvent to be used is not particularly limited, it is preferably about 5 ml to about 20 ml, particularly preferably about 9 ml to about 11 ml, per 1 g of the diester derivative (V).

The reaction temperature is between room temperature and 70°C, preferably 15°C to 35°C.

A heteroarylcarboxylic acid ester derivative (VI) or a salt thereof can be crystallized from the reaction system by filtering off the catalyst and adjusting the pH of the obtained filtrate with an aqueous alkaline solution such as sodium hydroxide and the like, and can be isolated and purified by filtration and separation. The pH of the filtrate is adjusted to 6 to 9, preferably 7 to 7.5.

The crystallization temperature is between 0°C and 20°C, preferably 10°C. The crystallization time is generally about 1 to about 16 hr.

The term "reaction system" means a reaction mixture after performing a reaction or a reaction mixture obtained by filtering an insoluble material (e.g., catalyst and the like) off from a reaction mixture.

A compound represented by the formula (II) can be produced from a compound represented by the formula (XIII) by the following method. 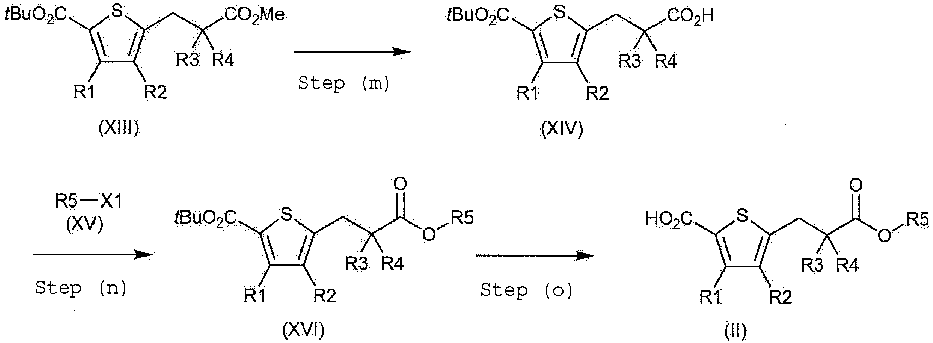 wherein X1 is a halogen atom such as a chlorine atom, a bromine atom and the like, and other symbols are as defined above.

### Step (m)

A compound represented by the formula (XIV) can be produced by subjecting a compound represented by the formula (XIII) to deprotection with a base such as sodium hydroxide and the like.

The amount of the base to be used is generally about 1 mol to about 2 mol, preferably about 1.6 mol, per 1 mol of compound (XIII).

As the reaction solvent, a mixed solvent of isopropyl alcohol and water is preferable.

The reaction temperature is between 10°C and 40°C, preferably 25°C. The reaction time is generally about 16 hr.

### Step (n)

A compound represented by the formula (XVI) can be produced by reacting a compound represented by the formula (XIV) with a compound represented by the formula (XV) in the presence of a base such as potassium carbonate and the like.

The amount of compound (XV) to be used is generally about 1 mol to about 2 mol, preferably about 1.1 mol, per 1 mol of compound (XIV).

The amount of the base to be used is generally about 1 mol to about 2 mol, preferably about 1.1 mol, per 1 mol of compound (XIV).

As the reaction solvent, N,N-dimethylformamide is preferable.

The reaction temperature is between 50°C and 70°C, preferably 60°C. The reaction time is generally about 2 hr.

### Step (o)

A compound represented by the formula (II) can be produced by subjecting a compound represented by the formula (XVI) to deprotection with an acid such as methansulfonic acid and the like.

The amount of the acid to be used is generally about 1 mol to about 3 mol, preferably about 2 mol, per 1 mol of compound (XVI).

As the reaction solvent, ethyl acetate is preferable.

The reaction temperature is between 50°C and 70°C, preferably 60°C. The reaction time is generally about 1 hr.

A compound represented by the formula (XIII) can be produced by the method described in WO 2013/187533.

A crystal of 3-[5-(4-carbamimidoyl-2-fluorophenoxy)carbonyl-2-thienyl]-2,2-dimethylpropanoic acid hydrochloride contains at least 19.6°, 23.1°, 24.0° and 24.2°(2θ), preferably at least 15.6°, 19.6°, 23.1°, 24.0°, 24.2°, 24.7° and 26.2° (2θ), in the peaks in a powder X-ray diffraction pattern.

The crystal of the present invention can be produced by the method described below.

3-[5-(4-Carbamimidoyl-2-fluorophenoxy)carbonyl-2-thienyl]-2,2-dimethylpropanoic acid is a compound represented by the following formula (X) (hereinafter to be also referred to as compound (X)).

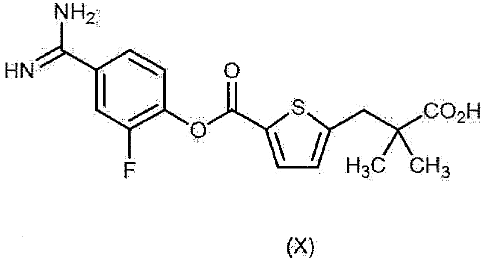

The crystal of the present invention can be produced by crystallizing hydrochloride of compound (X) in a solvent.

When a free form of compound (X) or a salt other than hydrochloride of compound (X) is used, crystallization is performed after conversion to hydrochloride.

Compound (X) or a salt thereof can be produced according to the production method of the compound of the formula (VI) of the present invention.

Compound (X) or a salt thereof also includes a solvate thereof, for example, hydrate, alcohol adduct and the like. As the alcohol adduct, methanol solvate, ethanol solvate, isopropyl alcohol solvate and the like can be mentioned.

As the salt of compound (X), salts with inorganic acids (e.g., hydrochloric acid, sulfuric acid, phosphoric acid, nitric acid, hydrobromic acid, etc.), organic carboxylic acids (e.g., carbonic acid, acetic acid, citric acid, benzoic acid, maleic acid, fumaric acid, tartaric acid, succinic acid, trifluoroacetic acid, tannic acid, butyric acid, decanoic acid, salicylic acid, lactic acid, oxalic acid, mandelic acid, malic acid, etc.), organic sulfonic acids (e.g., methanesulfonic acid, p-toluenesulfonic acid, benzenesulfonic acid, etc.) and the like can be mentioned.

Compound (X) or an alcohol adduct of compound (X) is dissolved or suspended in a solvent, and the solution or suspension is mixed with a hydrochloric acid or hydrogen chloride solution to give hydrochloride of compound (X).

As the solvent, a mixed solvent of alcohol such as methanol, ethanol, isopropyl alcohol and the like and water can be mentioned, and a mixed solvent of isopropyl alcohol and water is preferable. The ratio of isopropyl alcohol and water is preferably isopropyl alcohol:water = 1:1 to 1:10, more preferably 1:5 to 1:9, in a volume ratio. The amount of the solvent to be used is, preferably about 4 ml to about 30 ml, particularly preferably about 12 ml to about 21 ml, per 1 g of compound (X) or a salt thereof.

The amount of hydrochloric acid or hydrogen chloride to be used is generally about 1 mol to about 10 mol, preferably about 1 mol to about 6 mol, per 1 mol of compound (X) or a salt thereof.

The step for converting to hydrochloride is preferably performed at a temperature of 0°C to 60°C.

A crystal of the present invention can be produced by crystallizing hydrochloride of compound (X) in a solvent.

As a crystallization solvent, a mixed solvent of alcohol such as methanol, ethanol, isopropyl alcohol and the like and a solvent such as ethyl acetate, water and the like can be mentioned, and a mixed solvent methanol and ethyl acetate is preferable. The ratio of methanol and ethyl acetate is preferably methanol:ethyl acetate = 1:0.1 to 1:5, more preferably 1:0.5 to 1:2, in a volume ratio. The amount of the crystallization solvent to be used is preferably about 5 ml to about 25 ml, particularly preferably about 10 ml to about 20 ml, per 1 g of hydrochloride of compound (X). To dissolve hydrochloride of compound (X), a trace amount of water or methanol is preferably added.

By adding a solvent showing low solubility of hydrochloride of compound (X) while performing a concentration operation of the above-mentioned solution of hydrochloride of compound (X), the ratio of the solvent can be increased, whereby the hydrochloride of compound (X) can be crystallized. Preferable examples of the solvent include ethyl acetate. The amount of the solvent to be used is preferably about 15 ml to about 35 ml, particularly preferably about 20 ml to about 30 ml, per 1 g of hydrochloride of compound (X).

The hydrochloride of compound (X) can be crystallized by cooling the temperature of the obtained mixture at room temperature or 0°C to not more than room temperature.

A crystal of hydrochloride of compound (X) of the present invention can be obtained by isolating the crystal precipitated in the above.

The above-mentioned crystallization can also be performed in the same reaction mixture as in the step for converting to hydrochloride.

### Examples

While the present invention is explained in detail in the following by referring to Examples, the present invention is not limited to the following Examples.

### (Analysis conditions)

The analysis in the following Examples was performed using the following measuring apparatus and according to a conventional method.

### (1) ¹H-NMR

apparatus: Avance III 400, manufactured by Burker

### (2) high performance liquid chromatography (HPLC)

column: manufactured by GL Sciences Inc. InertSustaine 4.6 mm I.D.×150 mm, 3 µm
column temperature: 40°C
flow rate: 1.0 mL/min (Examples 1 to 3), 1.5 mL/min (Examples 4 to 6)
detection method: UV 254 nm
sample injection volume: 10 µL
analysis time: 30.0 min (Examples 1 to 3), 18.0 min (Examples 4 to 6)
eluent composition A: 0.1% TFA-water
B: 0.1% TFA-MeCN
gradient: B conc.% 0-4 min, 5%, 4-26 min, 5→90%, 26-34 min 90% (Examples 1 to 3)
gradient: B conic.% 0 min, 20%, 0-13 min, 20→90%, 13-15 min 90% (Examples 4 to 6)

In each step of the synthesis, the content value (%) of the obtained object product was obtained by comparison of HPLC area with that of reference standard.

### (Example 1)

Synthesis of (4-carbamimidoyl-2-fluorophenyl) 5-(3-benzyloxy-2,2-dimethyl-3-oxopropyl)thiophene-2-carboxylate trifluoroacetate

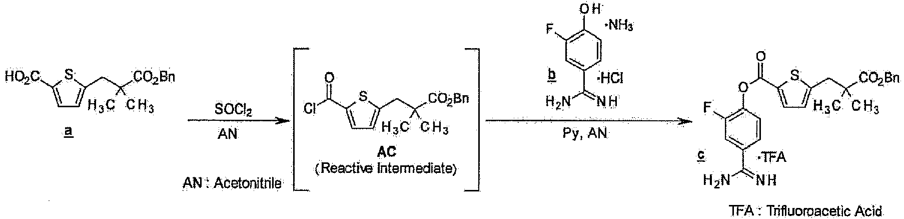

To a suspension of 5-(3-benzyloxy-2,2-dimethyl-3-oxopropyl)thiophene-2-carboxylic acid (35.0 g) in acetonitrile (AN, 130 mL) was added dropwise thionyl chloride (10.4 mL, 1.3 eq) with stirring at 20°C and, after the completion of the dropwise addition, the funnel was washed with AN (10.5 mL). After 3.5 hr, the reaction mixture was added dropwise to a suspension of ammonia-hydrogen chloride mixture of 3-fluoro-4-hydroxybenzamidine (25.1 g, 1.1 eq) and pyridine (26.7 mL, 3.0 eq) in AN (94.5 mL) with stirring at not more than 0°C and, after the completion of the dropwise addition, the funnel was washed with AN (10.5 mL). After 1 hr, water (245 mL) was added dropwise at not more than 0°C, trifluoroacetic acid (TFA, 25.4 mL, 3.0 eq)-water (123 mL) was added dropwise at not more than 10°C, and the mixture was stirred at 10°C overnight. The precipitated crystals were collected by filtration, washed with AN (112 mL)-water (168 mL), and dried under reduced pressure at 50°C to give the title compound (59.4 g) (content 97.6 wt%, yield 96.8%).
¹H-NMR (400MHz, DMSO-d6) δ 1.23 (6H, s), 3.20 (2H, s), 5.15 (2H, s), 7.02 (1H, d, J=3.6Hz), 7.32-7.40 (5H, m), 7.76-7.77 (2H, m), 7.93 (1H, d, J=3.6Hz), 7.94-7.97 (1H, d, J=1.6, 10.8Hz), 9.45 (2H, bs), 9.53 (2H, bs).

### (Example 2)

### Synthesis of 3-[5-(4-carbamimidoyl-2-fluorophenoxy)carbonyl-2-thienyl]-2,2-dimethylpropanoic acid isopropyl alcohol solvate

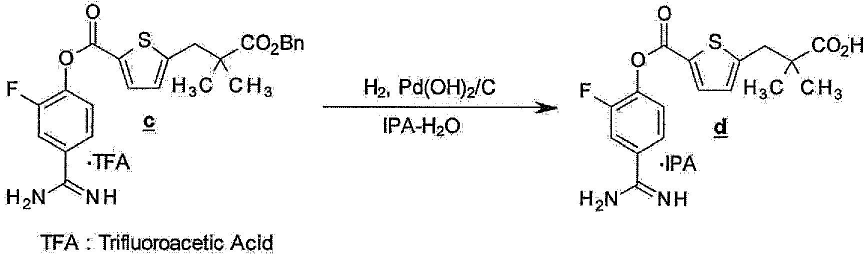

To a suspension of (4-carbamimidoyl-2-fluorophenyl) 5-(3-benzyloxy-2,2-dimethyl-3-oxopropyl)thiophene-2-carboxylate trifluoroacetate (49.0 g) in isopropyl alcohol (IPA, 353 mL)-Milli Q water (88 mL) was added 20% palladium hydroxide/carbon (about 50% water wet product, separately charged to about 0.35 eq in total), and the mixture was stirred under a hydrogen atmosphere at 25°C until HPLC Area% (starting material/object product) became 1.0% or below. Activated carbon was filtered off, and washed with IPA (78 mL)-water (20 mL), the obtained filtrate was cooled, 1 M sodium hydroxide (about 82 mL) was added dropwise at not more than 10°C, pH was adjusted to 7.4 and the mixture was stirred at 10°C overnight. The precipitated crystals were collected by filtration, washed with IPA (78 mL)-water (20 mL), and dried under reduced pressure at 50°C to give the title compound (34.5 g) (content 99.1 wt%, yield 93.3%).
¹H-NMR (400MHz, DMSO-d6, TFA) δ 1.04 (6H, d, J=6.0Hz), 1.16 (6H, s), 3.14 (2H, s), 3.79 (1H, m), 4.35 (1H, bs), 7.09 (1H, d, J=4.0Hz), 7.70-7.98 (4H, m), 9.43 (2H, bs), 9.74 (2H, bs), 12.57 (1H, bs).

### (Example 3)

### Synthesis of 3-[5-(4-carbamimidoyl-2-fluorophenoxy)carbonyl-2-thienyl]-2,2-dimethylpropanoic acid hydrochloride

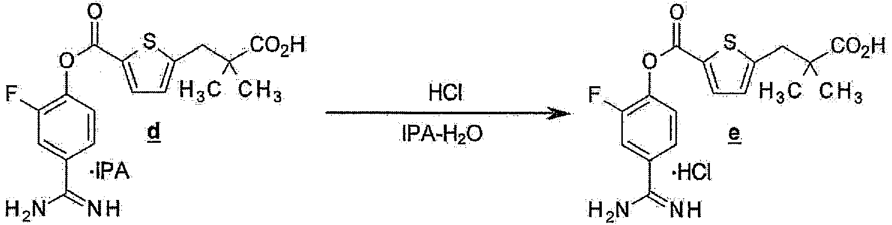

To a suspension of 3-[5-(4-carbamimidoyl-2-fluorophenoxy)carbonyl-2-thienyl]-2,2-dimethylpropanoic acid isopropyl alcohol solvate (27.8 g) in water (61 mL)-IPA (58 mL) was added dropwise 6 M hydrochloric acid (12.0 mL, 1.1 eq) with stirring at 20°C and, after the completion of the dropwise addition, the funnel was washed with water (6.7 mL). The mixture was heated to 45°C to dissolve same. After 2 hr, water (445 mL) was added dropwise and, after the completion of the dropwise addition, the mixture was cooled to 5°C over 16 hr. The precipitated crystals were collected by filtration, washed with IPA (11 mL)-water (100 mL), and dried under reduced pressure at 50°C to give the title compound (25.1 g) (content 99.9 wt%, yield 95.5%).
¹H-NMR (400MHz, DMSO-d6) δ 1.16 (6H, s), 3.14 (2H, s), 7.09 (1H, d, J=4.0Hz), 7.74-7.83 (2H, m), 7.97 (1H, d, J=3.6Hz), 8.01 (1H, dd, J=2.0Hz, J=10.8Hz), 9.49 (2H, bs), 9.59 (2H, bs), 12.57 (1H, bs).

### (Example 4)

### Synthesis of 3-(5-tert-butoxycarbonyl-2-thienyl)-2,2-dimethylpropanoic acid

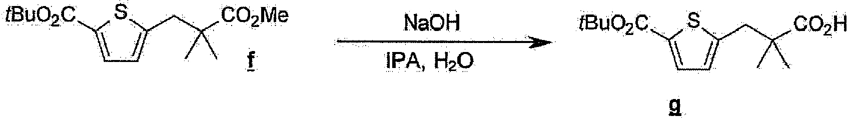

To an IPA solution (gross 54.2 g, net 16.0 g) of tert-butyl 5-(3-methoxy-2,2-dimethyl-3-oxopropyl)thiophene-2-carboxylate were added dropwise water (20.4 mL) and 6 M sodium hydroxide (11.6 mL), and the mixture was stirred at 25°C for 18 hr. To the reaction mixture was added dropwise 6 M hydrochloric acid (11.6 mL) at not more than 15°C, and the mixture was concentrated under reduced pressure. After a partitioning operation by adding ethyl acetate (80 mL) and water (48 mL), the organic layer was washed with water (48 mL). The obtained organic layer was concentrated to some extent, a seed crystal was added, the solvent was substituted with heptane, and the mixture was aged at 10°C overnight. The obtained suspension was filtered and the solid collected by filtration was washed with heptane (32 mL), and dried under reduced pressure at 40°C to give the title compound as a crude product (13.6 g) (content 84.2 wt%, yield 75.1%).

The crude product (13.6 g) was dissolved in IPA (29 mL) at 40°C, water (17.5 mL) was added at not less than 38°C, a seed crystal was added, and the mixture was stirred at 40°C for 1 hr. Water (40 mL) was further added at 40°C, and the mixture was stirred at 3.5 hr. The suspension was cooled to 10°C over 3 hr, and the mixture was aged overnight. The obtained crystals were collected by filtration, washed with water (18 mL), and dried under reduced pressure at 60°C to give the title compound (12.1 g) (content 96.9 wt%, yield 103%). ¹H-NMR (400MHz, CDCl3) δ 1.27 (6H, s), 1.56 (9H, s), 3.08 (2H, d, J=0.8 Hz), 6.79 (1H, d, J=3.6 Hz), 7.55 (1H, d, J=3.6 Hz).

### (Example 5)

### Synthesis of tert-butyl 5-(3-benzyloxy-2,2-dimethyl-3-oxopropyl)thiophene-2-carboxylate

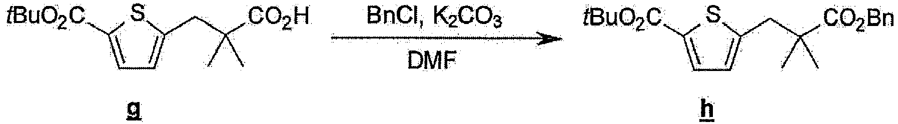

To a suspension of 3-(5-tert-butoxycarbonyl-2-thienyl)-2,2-dimethylpropanoic acid (11.8 g) obtained in Example 4, N,N-dimethylformamide (DMF, 23 mL) and potassium carbonate (5.56 g) was added dropwise benzyl chloride (5.10 mL), and the mixture was stirred at 60°C for 2.5 hr. The reaction mixture was filtered through celite, the solid was washed with ethyl acetate (23 mL), and a partitioning operation was performed by adding ethyl acetate (23 mL), water (58 mL) and sodium chloride (2.88 g) to the filtrate. Ethyl acetate (12 mL) was added to the organic layer, and the mixture was washed with water (35 ml), and washed again with water (23 mL). The obtained organic layer was concentrated under reduced pressure to give the title compound as a crude product (47.7 g) (content 30.4 wt%, yield 96.2%).

### (Example 6)

### Synthesis of 5-(3-benzyloxy-2,2-dimethyl-3-oxopropyl)thiophene-2-carboxylic acid

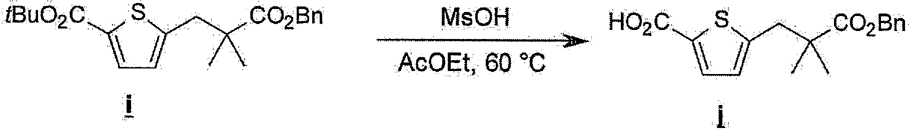

To tert-butyl 5-(3-benzyloxy-2,2-dimethyl-3-oxopropyl)thiophene-2-carboxylate (47.7 g) obtained in Example 5 was added dropwise methanesulfonic acid (5.0 mL) at room temperature, and the mixture was stirred at 60°C for not less than 30 min. The reaction mixture was stirred under temporary reduced pressure at 60°C for 1 hr, and a partitioning operation was performed by adding ethyl acetate (58 mL) and water (29 mL). The obtained organic layer was washed twice with water (29 mL), and concentrated under reduced pressure (63 g). A seed crystal was added, the solvent was substituted with heptane, and the mixture was aged at 10°C overnight. The obtained crystals were collected by filtration, washed with heptane (21 mL), and dried under reduced pressure at 40°C to give the title compound (12.0 g) (content 100 wt%, yield 97.8%).
¹H-NMR (400MHz, DMSO-d6) δ 1.18 (6H, s), 3.09 (2H, s), 5.12 (2H, s), 6.82 (1H, d, J=3.6Hz), 7.33-7.39 (5H, m), 7.53 (1H, d, J=3.6Hz), 12.94 (1H, s).

### (Example 7)

A powder X-ray diffraction pattern of the crystal of 3-[5-(4-carbamimidoyl-2-fluorophenoxy)carbonyl-2-thienyl]-2,2-dimethylpropanoic acid hydrochloride obtained by the method of Example 3 was measured.

The measurement conditions of the powder X-ray diffraction pattern were as follows.
Powder X-ray diffraction apparatus: X'Pert (manufactured by PANalytical B.V.)
Target: Cu full automatic monochromator
Voltage: 40 kV
Current: 30 mA (stability evaluation was performed at 55 mA) Slits: divergence slit 1/2°
scattering slit 1/2°
light receiving slit 0.15 mm
Scan Speed: 2°/min, 2θ range: 3 - 40°

The X-ray diffraction pattern chart is shown in Fig. 1.

### (Example 8)

A crystal of 3-[5-(4-carbamimidoyl-2-fluorophenoxy)carbonyl-2-thienyl]-2,2-dimethylpropanoic acid hydrochloride obtained by the method of Example 3 was preserved for 6 months under 40°C/75% RH conditions. As a result, water content and content (converted to a dehydrated form) did not show time-course changes, and the crystal was stable at 40°C/75% RH for 6 months. Therefore, the crystalline form is useful as a crystalline form of a drug substance for pharmaceutical products.

**Table 1**

| measurement item | start | 6 months |
|---|---|---|
| water content (%) | 0.1 | 0.1 |
| content (%) (converted into dehydrated form) | 99.5 | 99.5 |

The water content was measured by the Karl-Fischer method, and the content was measured by the HPLC method using a column charged with octadecylsilyl silica gel for liquid chromatography.

### Industrial Applicability

Using the production method of the present invention, the object compound, a heteroarylcarboxylic acid ester derivative, can be produced in a high yield and with high purity. Since a compound of the formula (II), a compound of the formula (VII), a compound of the formula (V) and a compound of the formula (VIII) are useful as intermediates for producing a heteroarylcarboxylic acid ester derivative (VI). Furthermore, since a crystal of a heteroarylcarboxylic acid ester derivative useful as a therapeutic drug for diabetes shows properties excellent in preservation stability, it is useful as a crystalline form of a drug substance for pharmaceutical products.

This application is based on patent application No. 2014-48019 filed in Japan, the entire contents of which are incorporated by reference herein.

## Claims

1. A method for puroducing a heteroarylcarboxylic acid ester derivative represented by the formula (VI) or a salt thereof: 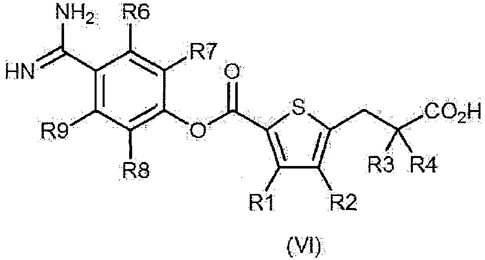
wherein R1 and R2 are the same or different and each is independently a hydrogen atom or a lower alkyl group optionally having a substituent(s),
R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto, and
R6, R7, R8 and R9 are the same or different and each is independently a hydrogen atom or a halogen atom,
the method comprising the following steps (d) to (f):
(d) a step of reacting a compound represented by the formula (II) with an acid halogenating agent to give an acid halide represented by the formula (III): 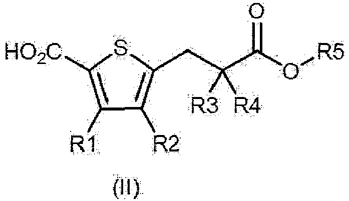 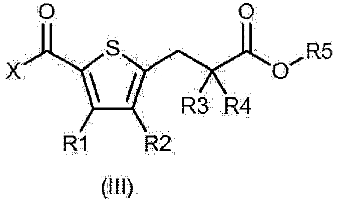 wherein R5 is an aralkyl group optionally having a substituent(s), an aralkyloxymethyl group optionally having a substituent(s), or a heteroarylalkyl group optionally having a substituent(s),
X is a halogen atom, and
other symbols are as defined above,
(e) a step of reacting the acid halide represented by the formula (III) with a compound represented by the formula (IV) in the presence of a base, and
crystallizing a compound represented by the formula (V) or a salt thereof from the reaction system: 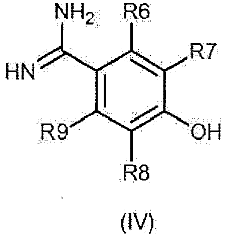 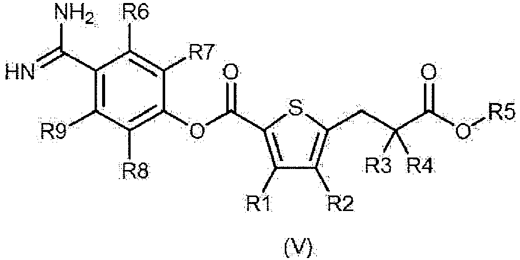 wherein each symbol is as defined above, and
(f) a step of subjecting the compound represented by the formula (V) to reductive deprotection in the presence of a metal catalyst, and crystallizing a heteroarylcarboxylic acid ester derivative represented by the formula (VI) or a salt thereof from the reaction system.

2. A method for producing a heteroarylcarboxylic acid ester derivative represented by the formula (VI) or a salt thereof: 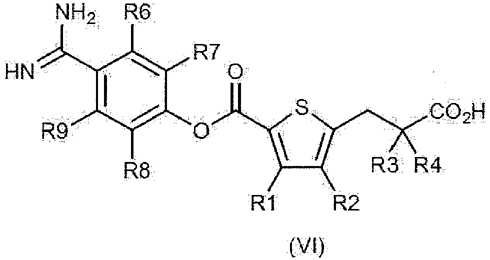
wherein R1 and R2 are the same or different and each is independently a hydrogen atom or a lower alkyl group optionally having a substituent(s),
R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto, and
R6, R7, R8 and R9 are the same or different and each is independently a hydrogen atom or a halogen atom,
the method comprising the following steps (g) to (i):
(g) a step of reacting a compound represented by the formula (II) with thionyl chloride or oxalyl chloride to give an acid chloride represented by the formula (IX): 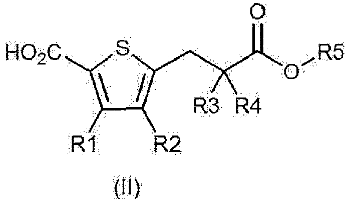 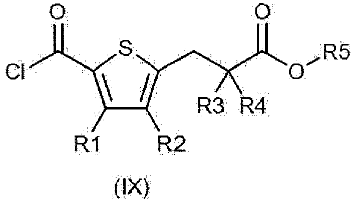 wherein R5 is an aralkyl group optionally having a substituent(s), an aralkyloxymethyl group optionally having a substituent(s), or a heteroarylalkyl group optionally having a substituent(s), and
other symbols are as defined above,
(h) a step of reacting the acid chloride represented by the formula (IX) with a compound represented by the formula (IV) in the presence of an organic base, and crystallizing a compound represented by the formula (V) or a salt thereof from the reaction system: 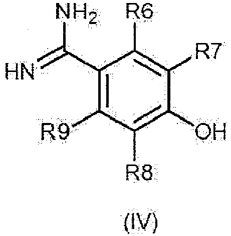 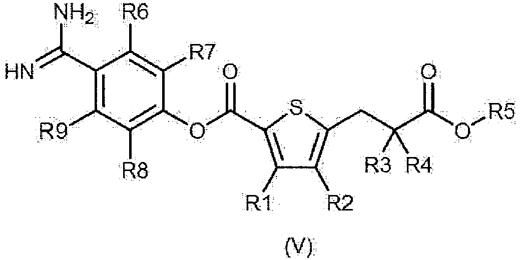 wherein each symbol is as defined above, and
(i) a step of subjecting the compound represented by the formula (V) to hydrogenolysis in the presence of a palladium catalyst, and crystallizing a heteroarylcarboxylic acid ester derivative represented by the formula (VI) or a salt thereof from the reaction system.

3. The method according to claim 1 or 2, wherein R5 is an aralkyl group optionally having a substituent(s).

4. The method according to claim 1 or 2, wherein R1 and R2 are hydrogen atoms, and R5 is an aralkyl group optionally having a substituent(s).

5. The method according to claim 1 or 2, wherein R3 and R4 are methyl groups, and
R5 is an aralkyl group optionally having a substituent(s).

6. The method according to claim 1 or 2, wherein R1 and R2 are hydrogen atoms,
R3 and R4 are methyl groups, and
R5 is an aralkyl group optionally having a substituent(s).

7. The method according to claim 1 or 2, wherein R1 and R2 are hydrogen atoms,
R3 and R4 are methyl groups,
R5 is a benzyl group,
R8 is a fluorine atom, and
R6, R7 and R9 are hydrogen atoms.

8. A method for puroducing a heteroarylcarboxylic acid ester derivative represented by the formula (X) or a salt thereof: 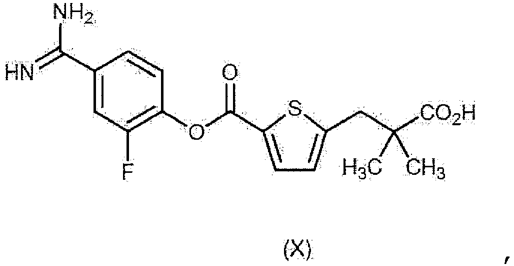 the method comprising the following steps (j) to (1) :
(j) a step of reacting a compound represented by the formula (VII) with thionyl chloride to give an acid chloride represented by the formula (XI): 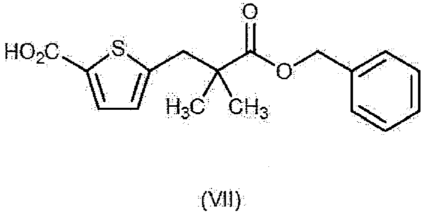 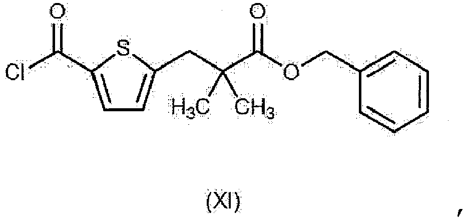
(k) a step of reacting the acid chloride represented by the formula (XI) with a compound represented by the formula (XII) in the presence of pyridine, and crystallizing a compound represented by the formula (VIII) or a salt thereof from the reaction system: 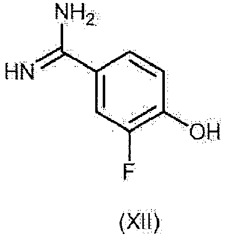 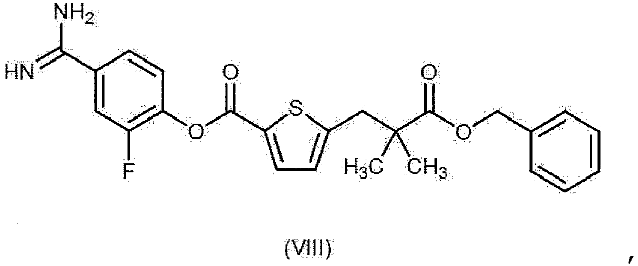 and
(1) a step of subjecting the compound represented by the formula (VIII) to hydorogenolysis in the presence of a palladium hydroxide catalyst, and crystallizing a heteroarylcarboxylic acid ester derivative represented by the formula (X) or a salt thereof from the reaction system.

9. A compound represented by the following formula (II), or a chemically acceptable salt thereof: 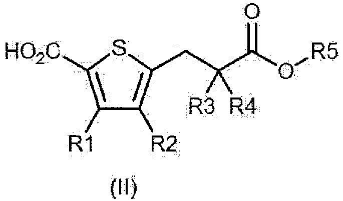
wherein R1 and R2 are the same or different and each is independently a hydrogen atom or a lower alkyl group optionally having a substituent(s),
R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto, and
R5 is an aralkyl group optionally having a substituent(s), an aralkyloxymethyl group optionally having a substituent(s), or a heteroarylalkyl group optionally having a substituent(s).

10. The compound according to claim 9, wherein, in the formula (II), R1 and R2 are hydrogen atoms,
R3 and R4 are the same or different and each is independently a methyl group, an ethyl group or a propyl group (excluding when R3 and R4 are ethyl groups), or R3 and R4 form a cyclopropane ring, a cyclobutane ring or a cyclopentane ring together with a carbon atom bonded thereto,
or a chemically acceptable salt thereof.

11. The compound according to claim 9 or 10, wherein, in the formula (II), R3 and R4 are methyl groups,
or a chemically acceptable salt thereof.

12. A compound represented by the following formula (VII), or a chemically acceptable salt thereof:

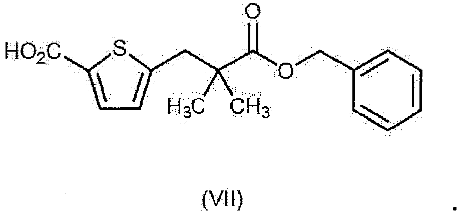

13. A compound represented by the following formula (V), or a chemically acceptable salt thereof: 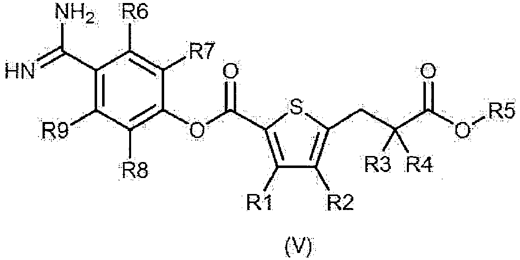
wherein R1 and R2 are the same or different and each is independently a hydrogen atom or a lower alkyl group optionally having a substituent(s),
R3 and R4 are the same or different and each is independently a hydrogen atom or a lower alkyl group optionally having a substituent(s) (excluding when R3 and R4 are ethyl groups), or R3 and R4 form a C₃₋₈ cycloalkane ring together with a carbon atom bonded thereto,
R5 is an aralkyl group optionally having a substituent(s), an aralkyloxymethyl group optionally having a substituent(s), or a heteroarylalkyl group optionally having a substituent(s), and R6, R7, R8 and R9 are the same or different and each is independently a hydrogen atom or a halogen atom.

14. The compound according to claim 13, wherein, in the formula (V), R1 and R2 are hydrogen atoms, and
R3 and R4 are the same or different and each is independently a methyl group, an ethyl group or a propyl group (excluding when R3 and R4 are ethyl groups), or R3 and R4 form a cyclopropane ring, a cyclobutane ring or a cyclopentane ring together with a carbon atom bonded thereto,
or a chemically acceptable salt thereof.

15. The compound according to claim 13 or 14, wherein, in the formula (V), R3 and R4 are methyl groups, or a chemically acceptable salt thereof.

16. A compound represented by the following formula (VIII), or a chemically acceptable salt thereof:

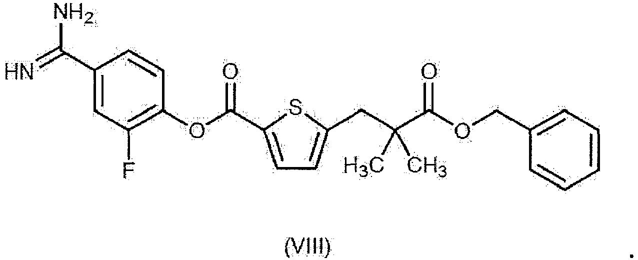

17. A crystal of 3-[5-(4-carbamimidoyl-2-fluorophenoxy)carbonyl-2-thienyl]-2,2-dimethylpropanoic acid hydrochloride showing an X-ray powder diffraction pattern comprising peaks at least at 19.6°, 23.1°, 24.0° and 24.2° (2θ).
